# EUROPÄISCHE PATENTSCHRIFT

(11) **EP 1 701 748 B1**
(45) Veröffentlichungstag und Bekanntmachung des Hinweises auf die Patenterteilung: **01.10.2008**
(21) Anmeldenummer: 04820060.4
(22) Anmeldetag: 09.12.2004
(51) Int. Cl.: A61L 9/22, F24F 3/16, F24F 13/068, E04B 9/02

(54) **ANORDNUNG ZUR BEEINFLUSSUNG UND BEHANDLUNG DER LUFT WENIGSTENS EINES RAUMES**
SYSTEM FOR INFLUENCING AND TREATING THE AIR OF AT LEAST ONE ROOM
INSTALLATION POUR TRAITER ET INFLUER SUR L'AIR D'AU MOINS UNE PIECE

(30) Priorität: 12.12.2003 DE 10360237; 31.03.2004 JP 2004104367
(43) Veröffentlichungstag der Anmeldung: 20.09.2006
(73) Patentinhaber: LK Luftqualität AG, 6015 Reussbühl (CH)
(72) Erfinder: FLEISCHER, Werner, CH-6103 Schwarzenberg (CH)
(74) Vertreter: Uhlemann, Henry
(86) Internationale Anmeldenummer: PCT/EP2004/014027
(87) Internationale Veröffentlichungsnummer: WO 2005/056065

(56) Entgegenhaltungen:
- EP-A- 0 756 138
- EP-A- 1 125 588
- DE-A1- 4 421 167
- DE-A1- 10 007 523
- US-A- 5 259 553

## Beschreibung

Die Erfindung betrifft Anordnungen zur Beeinflussung und Behandlung der Luft wenigstens eines Raumes durch sowohl Temperieren als auch Ionisation der Zuluft.

Bekannterweise werden mit Ionisierungsapparaten die Raumluft und damit die Atemluft behandelt. Dabei werden Bakterien und andere Keime abgetötet und Großmoleküle in kleinmolekulare Fragmente aufgespalten. Komplexe und große Moleküle sind unter anderem auch Geruchsstoffe, so dass mit einer Luftionisation eine Geruchsbelastung unterdrückt werden kann. Es können weiterhin sowohl sogar gesundheitlich schädliche Lastsituationen in der Raumluft eliminiert als auch Mikroorganismen in der Luft wirksam reduziert werden.

In Ionisationsapparaten werden elektrische Felder zwischen zwei Elektroden mit Spannungspotenzialen ausgenutzt, um durch Gasentladungen Ionen durch Stoßionisationen zu erzeugen. Dazu werden bekannterweise Ionisierungsröhren in Form von Glasröhren eingesetzt, bei denen die Innenseite beschichtet und die Außenseite elektrisch leitend sind. Dazu befinden sich bekannterweise vorzugsweise rohrförmige Metallgitter auf der Außenseite der Glasröhren, so dass ein koaxialer Aufbau vorhanden ist. Wird eine für die Gasentladung ausreichend hohe elektrische Spannung angelegt, bildet das Glas der Wandung ein Dielektrikum, in dem ein großes elektrisches Feld vorhanden ist. Die strömende Luft wird mit Ionen angereichert. In der DE 196 51 402 A1 (Apparat zur physikalischen Aufbereitung von Luft, insbesondere von Atemluft) werden Luftionisatoren flacher Bauart als Elektroden eingesetzt, die beabstandet voneinander angeordnet sind.

Ein wesentlicher Nachteil dieser Anordnungen besteht darin, dass es ab einer bestimmten Spannung zu einer Bildung von Ozon kommt, die sich mit der Vergrößerung der Spannung erhöht.

In der DE 43 34 956.0 C2 (Verfahren zur Luftbehandlung mit Ionen sowie Vorrichtung zur Durchführung des Verfahrens) sind ein Verfahren zur Luftbehandlung mit Ionen und eine Vorrichtung zur Durchführung des Verfahrens beschrieben, wobei die Langzeitstabilität eines Ionisationsapparates erhöht wird. Dabei wird die Entladungsspannung so gesteuert, dass eine erhöhte Ozonerzeugung nicht erfolgt. Wie bei unbelasteter natürlicher Luft wird technisch mit dem vorbeschriebenen Verfahren und der vorbeschriebenen Vorrichtung sichergestellt, dass ständig Sauerstoffionen vorhanden sind. Mittels der verwendeten Sensoren, in Form eines Luftqualitätssensors, eines Luftströmungsfühlers und eines Luftfeuchtefühlers kann die Einhaltung dieser Mindestintensität in einem Lastbereich im Wesentlichen eingehalten werden.

Beim Auftreten sowohl äußerer Störquellen wie zum Beispiel bei Smog, einer Inversionswetterlage, Gewitter, äußere Energiefelder als auch innerer Störquellen durch den Betrieb elektrischer Geräte kann die Belastung an Ozon in der Zuluft in unerwünschtern Maß ansteigen und zu einer Grenzwertüberschreitung fuhren. In der DE 100 07 523 C2 (Verfahren zur Luftbehandlung mit Ionen sowie Vorrichtung zur Durchführung des Verfahrens) ist deshalb zusätzlich ein Ozonsensor zur Bestimmung des Ozongehaltes ein Bestandteil dieser Vorrichtung. Über das Steuergerät wird die Ionisationsleistung auch hinsichtlich des Ozongehalts so gesteuert, dass sogar ein Ozonabbau gegeben ist.

In der EP 1 078 205 B1 (Luftkühlelement, Verfahren zu seinem Betrieb sowie Luftkühlanordnung) wird ein Lüftkühlelement mit einer Kammer die mindestens einen Lufteinlass aufweist und einseitig von einer Kühlwand begrenzt und im übrigen im wesentlichen luftdicht verschlossen ist, beschrieben. Die Kühlwand besitzt über ihre Fläche verteilte Mikrolöcher. Diese Lösung ist nur für das Temperieren eines Raumes vorgesehen. Weiterhin sind mehrere derartige Kammern für einen Raum notwendig, so dass zwischen benachbart angeordneten Kammern sichtbare Fugen vorhanden sind. Damit sind derartig ausgebildete Decken insbesondere für Wohnräume wenig geeignet. Ein weiterer Nachteil stellen die Mikrolöcher dar, wobei während einer Beschichtung der Kühlwände diese Mikrolöcher nicht verschlossen werden dürfen. Um das zu gewährleisten sind aufwändige Technologien notwendig, so dass derartige Kammern ökonomisch nicht einfach realisierbar sind.

Durch die US 5 259 553 A (interior atmosphere control system) ist es bekannt, um die Zuluft zu temperieren, eine Vorrichtung in der Zuluftleitung anzuordnen. Diese Vorrichtung ist dazu mit einer Steuereinrichtung zusammengeschaltet. Eine Beeinflussung der Luft hinsichtlich der Erhöhung der Luftqualität ist nicht vorgesehen.

Durch die EP 0 756 138 A (Flächiges Kühl- und Heizelement) ist ein flächiges Kühl- und Heizelement bekannt, dessen Innenraum mit einer temperierbaren Luftströmung beaufschlagbar ist und das zu dem Raum hin von einer porösen Deckschicht abgeschlossen ist. Dabei handelt es sich aber nur um ein Element, dessen Fläche aber nur einen kleinen Bereich zur Gesamtfläche des Raumes bildet. Nachteilig ist der Sachverhalt, dass sich die Aufenthaltszone des Menschen nicht darunter befinden soll.

Der im Patentanspruch 1 angegebenen Erfindung liegt die Aufgabe zugrunde, wenigstens einen Raum komfortabel zu klimatisieren, wobei sowohl eine hohe Luftqualität als auch eine zugfreie und damit komfortable Lufteinführung in den Raum gewährleistet sind.

Diese Aufgabe wird mit den im Patentanspruch 1 aufgeführten Merkmalen gelöst.

Die Anordnungen zur Beeinflussung und Behandlung der Luft wenigstens eines Raumes durch sowohl Temperieren als auch Ionisation der Zuluft zeichnen sich insbesondere durch die Gewährleistung einer hohen Luftqualität bei gleichzeitiger zugfreien und damit komfortablen Lufteinführung aus.

Die hohe Luftqualität wird durch eine gesteuerte Ionisation der Zuluft des wenigstens einen Raumes gewährleistet. Die Steuerung dieser Ionisation basiert auf den Messungen
- eines ersten Luftqualitätssensors in der Außenluftleitung eines Luftaufbereitungsgerätes,
- eines Ozonsensors, eines Luftfeuchtefühlers und eines Luftströmungsfühlers in der Zuluftleitung zwischen Luftaufbereitungsgerät und dem wenigstens einem Raum und
- eines zweiten Luftqualitätssensors in der Umluftleitung zwischen dem wenigstens einem Raum und dem Luftaufbereitungsgerät.

Eine Vorrichtung zum Lufttemperieren gewährleistet die Raumtemperatur, wobei sowohl eine Heizung als auch eine Kühlung gegeben ist. Damit ist eine Klimatisierung des wenigstens einen Raumes gewährleistet.

Die zugfreie und damit komfortable Lufteinführung basiert vorteilhafterweise auf kammerartigen und ein Bestandteil des Raumes bildenden Einrichtungen, die an die Zuluftleitung des wenigstens einen Raumes gekoppelt sind und wobei eine die kammerartigen Einrichtungen und den Raum voneinander trennende Wand die Konvektion der temperierten und durch Ionisation beeinflussten Zuluft in den Raum gewährleistende Öffnungen aufweist. Diese Öffnungen sind so ausgeführt, dass die temperierte und durch die Ionisation beeinflusste Luft ohne für Menschen spürbaren Zug und ohne wahrnehmbare Geräusche in den Raum strömen kann. Dadurch erfolgt die Beeinflussung der Raumluft durch Strahlungswärme der Fläche mit den Öffnungen und über Konvektion, wobei durch die Öffnungen eine hohe aber für Personen im Raum nicht fühl- und wahrnehmbare Luftströmung als laminarer Verdrängungsluftstrom vorhanden ist. Dadurch ist vorteilhafterweise ein kompakter Verdrängungsstrom der ionisierten Luft vorhanden. Das Mischungsverhältnis der Raumluft und der Zuluft wird mit Injektion der Zuluft erreicht. Dabei ist eine temperierte und beeinflusste Luftschicht an der Wand in Richtung des Raumes vorhanden. Diese Luftströmung und diese Luftschicht verhindert vorteilhafterweise, dass mit luftgetragenen Partikeln versehene und feuchte Raumluft an die Wand mit den Öffnungen gelangen kann. Sowohl das Ablagern und Ansammeln von Partikeln als auch die Ausbildung eines das Wachstum biologischer Partikel unterstützendes Mikroklima wird weitestgehend verhindert. Damit kann die erfindungsgemäße Anordnung über einen längeren Zeitraum ohne Reinigung betrieben werden.

Die kammerartigen Einrichtungen sind in einer Ebene angeordnet, wobei die in Richtung des Raumes angeordneten Kammerwände Durchbrüche aufweisen. Damit sind baulich begrenzte und leicht montierbare Einheiten vorhanden, in denen weiterhin die Zuluft ohne große Druckunterschiede gleichmäßig verteilbar ist. Die die Konvektion der Zuluft gewährleistenden Öffnungen sind Bestandteile eines auf den Kammerwänden oder beabstandet zu den Kammerwänden (siehe Anspruch 1) angeordneten Körpers. Damit können insbesondere fugenfreie und durchgängige Wände realisiert werden. Dabei wird vorteilhafterweise der Körper nach der Montage der kammerartigen Einrichtungen aufgebracht oder angeordnet.

Die Querschnitte der Durchbrüche als Öffnungen der kammerartigen Einrichtungen sind kleiner oder gleich als die Querschnitte der Öffnungen des Körpers. Dadurch ist kein Staudruck zwischen den kammerartigen Einrichtungen und dem beabstandet dazu angeordneten Körper vorhanden.

Vorteilhafterweise besteht zwischen der Zuluft und der Raumluft eine hohe Temperaturdifferenz, so dass dieser Vorgang und Zustand vorteilhaft unterstützt wird. Gleichzeitig ist damit eine geringe Luftmenge zur Klimatisierung notwendig. Dadurch können ökonomisch günstig auch mehrere Räume an ein zentral angeordnetes System zur Luftbeeinflussung gekoppelt werden. Damit eignet sich die erfindungsgemäße Anordnung insbesondere für Gebäude mit mehreren Arbeits- und/oder Wohnräumen, deren Raumhöhe keinen Einfluss auf die Klimatisierung hat. Die Anordnung zeichnet sich dabei weiterhin dadurch aus, dass diese auch nachträglich in bestehende Gebäude eingebaut werden kann.

Ein weiterer Vorteil der erfindungsgemäßen Anordnungen besteht darin, dass die Höhe der lonisationsleistung mindestens eines oder mehrerer Ionisationsapparate mit der Steuereinrichtung so geregelt wird, dass bei Auftreten eines zu hohen Wertes an Ozon dieses durch die Bildung freier Radikale als auch natürlicher Sauerstoffcluster als Zusammenballung elektrisch geladener Sauerstoffionen zurückgeführt wird. Dabei wird der Wert des Ozons in der Zuluft als Gemisch von Außen- und Umluft durch den Ozonsensor gemessen. Über die mit dem Ozonsensor verbundene Steuereinrichtung und darin festlegbare oder festgelegte Grenzwerte wird der Ionisationsapparat so beeinflusst, dass eine auf das Vorhandensein von Ozon zurückzuführende schädliche Wirkung auf im Raum sich aufhaltende Personen weitestgehend vermieden wird. Die Steuereinrichtung liefert für eine tatsächlich stabile und der Natur adäquate Zuluftionisation, wobei ein vorgegebener Ozongrenzwert nicht überschritten sowie bei einer Extremsituation Ozon eliminiert werden, optimierte Wechselimpulse, die zu dem mindestens einen Ionisationsapparat geleitet werden. Jeder Wechselimpuls ist eine volle Sinuskurve, die im Nulldurchgang, das bedeutet beim Wechsel der Halbwellen, angeschnitten sind (Phasenanschnittsteuerung). Die Frequenz wird dabei nicht verändert. Vorteilhafterweise werden mehrere Wechselimpulse als mehrere Sinuskurven zu Paketen zusammengefasst. Die Paketgröße und damit die Anzahl der Wechselimpulse je Paket stellt eine Möglichkeit dar, um die Luftionisation zu optimieren und gleichzeitig die Belastung der elektrischen Stromversorgung der erfindungsgemäßen Anordnung zu minimieren. Die Entladungsspannung bleibt dabei konstant, so dass eine stabile Luftionisation gewährleistet wird. Damit ist insbesondere eine gezielte Nutzung der Umluft gegeben, so dass insbesondere bei hohen Temperaturen im Sommer und bei niedrigen Temperaturen im Winter Energiekosten für das Kühlen oder Heizen eingespart werden.

Ein weiterer Vorteil der erfindungsgemäßen Anordnungen besteht darin, dass mit der Ionisation der Zuluft gasförmige flüchtige Kohlenwasserstoffe abgebaut, die Oxidationsmöglichkeit der Luft gesenkt und Mikroorganismen eliminiert werden.

Vorteilhafte Ausgestaltungen der Erfindung sind in den Patentansprüchen 2 bis 10 angegeben.

Über einen Regler für den Volumenstrom der Zuluft in der Zuluftleitung vor den kammerartigen Einrichtungen oder einer der kammerartigen Einrichtungen nach der Weiterbildung des Patentanspruchs 2 kann ein komfortables Klima im Raum manuell eingestellt und automatisch beibehalten werden. Insbesondere betrifft das die Temperatur im Raum. Dazu ist der Regler für den Volumenstrom und/oder die Steuereinrichtung und/oder eine weitere Steuerinrichtung mit einem im Raum angeordneten Temperatursensor verbunden. Über ein verstellbares Element, das mit einer weiteren Steuereinrichtung und/oder der Steuereinrichtung und/oder dem Regler verbunden ist, wird der Volumenstrom entsprechend des gewählten Wertes geregelt. Das Element ist dabei vorteilhafterweise entweder ein Potentiometer als stufenlos verstellbarer Spannungsteiler oder ein Schalter in Verbindung mit Widerständen als in Stufen verstellbarer Spannungsteiler.

Ein flexibler bahnenförmiger Körper, der die kammerartigen Einrichtungen überspannt, nach der Weiterbildung des Patentanspruchs 3 führt vorteilhafterweise dazu, dass einfach eine durchgehende Decke realisierbar ist. Unterbrechungen oder Abschnitte sind nicht sichtbar. Der flexible bahnenförmige Körper ist leicht als Ganzes abnehm- oder austauschbar, so dass dieser leicht gereinigt werden kann. Mit einem Austausch kann auch die Ästhetik des damit ausgerüsteten Raumes leicht geändert werden. Mit einem beabstandet zu den kammerartigen Einrichtungen angeordneten flexiblen bahnenförmigen Körper können weiterhin leicht Unebenheiten sowohl der Decke als auch der kammerartigen Einrichtungen ausgeglichen werden.

Die kammerartigen Einrichtungen führen vorteilhafterweise dazu, dass ein weitestgehend gleichmäßiges Temperieren oder Beeinflussen der Raumluft insbesondere auch bei großflächigen Räumen erfolgen kann. Dazu strömt die temperierte und beeinflusste Zuluft in diese Einrichtungen und über die Öffnungen des flexiblen bahnenförmigen Körpers in den Raum. Vorteilhafterweise kann diese Zuluft durch geeignete Einbauten in den kammerartigen Einrichtungen auch verwirbeln.

Dadurch erfolgt die Beeinflussung der Raumluft durch Strahlungswärme der Fläche mit den Öffnungen und über Konvektion, wobei durch die Öffnungen eine hohe aber für Personen im Raum nicht fühl- und wahrnehmbare Luftströmung vorhanden ist. Das Mischungsverhältnis der Raumluft und der Zuluft kann mit der Injektion der Zuluft erreicht werden. Dadurch ist eine temperierte und beeinflusste Luftschicht an der Wand in Richtung des Raumes vorhanden. Diese Luftströmung und diese Luftschicht verhindert vorteilhafterweise, dass mit luftgetragenen Partikeln versehene und feuchte Raumluft an die Wand mit den Öffnungen gelangen kann. Sowohl das Ablagern und Ansammeln von Partikeln als auch die Ausbildung eines das Wachstum biologischer Partikel unterstützendes Mikroklima wird weitestgehend verhindert. Damit kann die erfindungsgemäße Deckenanordnung über einen längeren Zeitraum ohne Reinigung eingesetzt werden.

Die Deckenanordnung zeichnet sich dabei vorteilhafterweise weiterhin dadurch aus, dass diese auch nachträglich in bestehende Gebäude leicht eingebaut werden kann.

Nach der Weiterbildung des Patentanspruchs 4 ist der flexible bahnenförmige Körper eine aus Kunststoff bestehende Folie oder besteht aus Faserstoffen. Dabei ist der flexible bahnenförmige Körper ein Gewebe, ein Gewirk, ein Gestrick oder ein Vlies. Derartige Körper sind auch bekannte Textilien.

Derartige Körper können insbesondere auch unterschiedlich farbig ausgestaltet sein, so dass gleichzeitig dieser Körper ein Dekorationselement des Raumes ist.

Um den Brandschutz in Gebäuden zu gewährleisten, besteht nach der Weiterbildung des Patentanspruchs 5 der Körper oder der flexible bahnenförmige Körper aus einem schwer oder nicht entflammbaren Material.

Eine günstige Realisierung zur Bestimmung der Höhe der Ionisationsleistung des Ionisationsapparates, wobei die Ionisation durch elektrische Entladung an Ionisationsröhren oder an Koronaentladungsröhren erfolgt, ist nach der Weiterbildung des Patentanspruchs 6 durch die Messungen insbesondere der Belastung der Außenluft mit flüchtigem Kohlenwasserstoff mit dem ersten Luftqualitätssensor, der Strömungsgeschwindigkeit oder des Volumenstroms der zu behandelnden Luft mit dem Luftströmungsfühler, der relativen Luftfeuchte der zu behandelnden Luft mit dem Luftfeuchtefühler, den Gehalt an Ozon in der Zuluft mit dem Ozonsensor und der oxydierbaren Luftbestandteile der Abluft und/oder Umluft mit dem zweiten Luftqualitätssensor gegeben.

Der Ionisationsapparat wird nach der Weiterbildung des Patentanspruchs 7 so betrieben, dass eine Mindestintensität an Sauerstoffionen entsprechend natürlichen Verhältnissen gewährleistet ist. Dazu wird der Ionisationsapparat ständig betrieben, so dass die dem Raum zuströmende Luft ständig beeinflusst wird. Bei sich plötzlich ändernden Bedingungen, zum Beispiel durch viele Raucher im Raum oder stark wirkende Reinigungsmittel oder sich erhöhenden Belastungen der Außenluft, ist die Zeitkonstante bis zu einer wirkungsvollen Ionisation wesentlich geringer, so dass die Raumluft schneller positiv beeinflusst oder sofort neutralisiert wird.

Die Ionisationsleistung des wenigstens einen Ionisationsapparates wird nach der Weiterbildung des Patentanspruchs 8 so gesteuert, dass diese bei sich erhöhenden Anteilen an flüchtigen Kohlenwasserstoffen und/oder Steigerung der Luftgeschwindigkeit und/oder Steigerung der relativen Luftfeuchte und/oder erhöhenden Anteil an oxidierbaren Luftbestandteilen steigt. Damit ist sichergestellt, dass sich bei verschlechternden Eigenschaften der Luftqualität im Raum weitestgehend unbelastete Zuluft durch den vorgebenen Luftwechsel und der optimierten Intensität in den Raum oder eine Aufenthaltszone gelangt.

Eine günstige Steuerung des Ionisationsapparates ist nach der Weiterbildungen des Patentanspruchs 9 über eine zeitlich anliegende periodische Wechselspannung gegeben. Dabei wird der Ionisationsapparat mit einem Wechselimpuls, Wechselimpulsen oder zu Paketen zusammengefassten Wechselimpulsen einer zur Verfügung stehenden periodischen Wechselspannung beaufschlagt. Vorteilhafterweise ist die optimierte Entladungsspannung dabei konstant.

Durch die Weiterbildung des Patentanspruchs 10 wird der Anteil an Ozon so gesenkt, dass die gewünschten und vorgegebenen Grenzwerte eines komfortablen Klimas im Raum gewährleistet werden. In einem ersten Bereich wird dazu die Leistung des Ionisationsapparates gesenkt. Steigt der Wert des Ozongehalts der Zuluft trotz der Absenkung der Luftionisation, so ist mindestens eine externe Ozonquelle vorhanden. In diesem Fall wird automatisch ein Modus zum Abbau des Ozones durch die Steuereinrichtung geschaltet. Werden die vorgegebenen Grenzwerte wieder erreicht, wird der Ionisationsapparat wieder auf Normalbetrieb geschaltet. Dabei wird das Energieniveau des Ozons so verändert, dass es zerfällt. Die Werte zur Signalisierung werden so gewählt, dass genügend Reaktionssicherheit besteht.

Ein Ausführungsbeispiel der Erfindung ist in den Zeichnungen dargestellt und wird im folgenden näher beschrieben.

Es zeigen:
- Fig. 1: eine prinzipielle und schematische Darstellung einer Anordnung zur Beeinflussung und Behandlung der Luft wenigstens eines Raumes,
- Fig. 2: eine prinzipielle Darstellung eines Wechselimpulsens zur Steuerung eines Ionisationsapparates der Anordnung,
- Fig.3: eine Deckenanordnung für einen temperierbaren Raum und/oder für die Beeinflussung der Eigenschaften der Luft eines Raumes in einer prinzipiellen Teilansicht in einer Ecke des Raumes,
- Fig. 4: eine Spann- und Befestigungseinnchtung zum lösbaren Spannen eines flexiblen bahnenförmigen Körpers,
- Fig. 5: Bestandteile einer Spann- und Befestigungseinrichtung zum lösbaren Spannen eines flexiblen bahnenförmigen Körpers in ihrem Profil und
- Fig. 6: eine weitere Spann- und Befestigungseinrichtung zum lösbaren Spannen eines flexiblen bahnenförmigen Körpers.

Eine Anordnung zur Beeinflussung und Behandlung der Luft wenigstens eines Raumes 4 ist im wesentlichen eine Kombination von Einrichtungen sowohl zum Temperieren als auch zur Ionisation der Zuluft des wenigstens eines Raumes 4.

In der Außenluftleitung 7 eines Luftaufbereitungsgerätes 1 befindet sich ein erster Luftqualitätssensor 12. In der Zuluftleitung 8 zwischen dem Luftaufbereitungsgerät 1 und dem wenigstens einem Raum 4 sind mindestens ein Ionisationsapparat 2, ein Ozonsensor 13, ein Luftfeuchtefühler 14, ein Luftströmungsfühler 15 und wenigstens eine Vorrichtung 3 zum Lufttemperieren eingeschaltet An die Zuluftleitung 8 sind mehrere kammerartige und ein Bestandteil des Raumes 4 bildende Einrichtungen 5 angekoppelt. An die Abluftleitung 9 des wenigstens einen Raumes 4 schließt sich sowohl eine nach außen endende Fortluftleitung 10 als auch eine mit dem Luftaufbereitungsgerät 1 verbundene Umluftleitung 11 an. In der Umluftleitung 11 ist ein zweiter Luftqualitätssensor 16 angeordnet. Der erste Luftqualitätssensor 12, der Ozonsensor 13, der Luftfeuchtefühler 14, der Luftströmungsfühler 15 und der zweite Luftqualitätssensor 16 sind mit wenigstens einer Steuereinrichtung 6 zusammengeschaltet. Die Steuereinrichtung 6 ist weiterhin mit dem Ionisationsapparat 2 und der Vorrichtung 3 zum Lufttemperieren verbunden. In der Zuluftleitung 8 vor der oder einer ersten kammerartigen Einrichtung 5 ist ein Regler 17 für den Volumenstrom der Zuluft eingeschalten. Der Antrieb des Stellelementes des Reglers 17 ist weiterhin mit der Steuereinrichtung 6 und/oder einer weiteren Steuereinrichtung für die Raumtemperatur zusammengeschaltet.

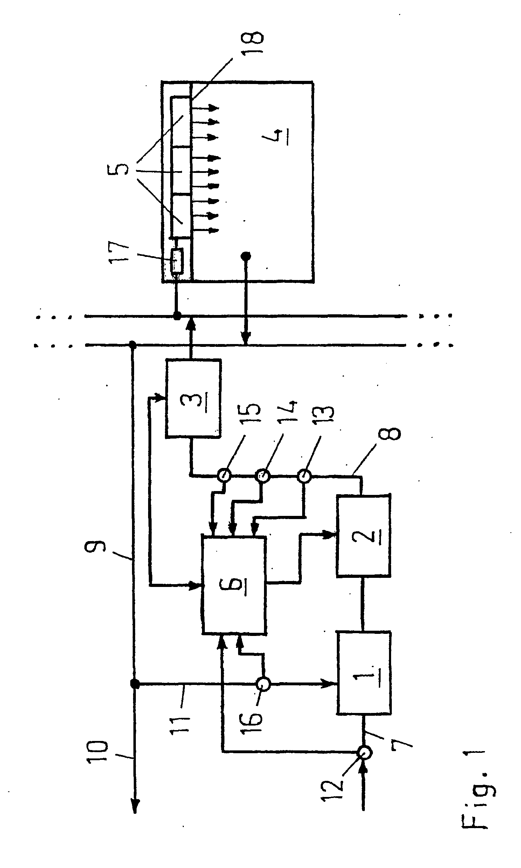

Die Fig. 1 zeigt eine derartige Anordnung zur Beeinflussung und Behandlung der Luft wenigstens eines Raumes 4 in einer prinzipiellen und schematischen Darstellung.
Die Vorrichtung 3 zum Lufttemperieren ist eine bekannte Heizeinrichtung und/oder Kühleinrichtung für Luft.

Mehrere kammerartige Einrichtungen 5 sind in einer Ebene an der Decke des Raumes 4 angeordnet. Die Hohlräume der kammerartigen Einrichtungen 5 sind über Rohrleitungen miteinander so verbunden, dass über die Zuluftleitung 8 nach der Vorrichtung 3 zum Lufttemperieren die Zuluft in diese kammerartigen Einrichtungen 5 strömen kann. Die in Richtung des Raumes weisenden Kammerwände der Einrichtungen 5 besitzen mehrere Öffnungen, im folgenden als Durchbrüche bezeichnet. An oder beabstandet zu diesen Kammerwänden ist ein Körper 18 angeordnet, der die Konvektion der temperierten und durch Ionisation beeinflussten Zuluft in den Raum 4 gewährleistende Öffnungen aufweiset. Diese Kammerwände und der lose angeordnete Körper 18 sind eine Wand des Raumes 4 und bilden damit eine Zwischendecke des Raumes 4.

Die Querschnitte der Durchbrüche als Öffnungen der kammerartigen Einrichtungen 5 sind kleiner oder gleich der Querschnitte der Öffnungen des Körpers 18. Insbesondere bei gegenüber den Durchbrüchen größeren Öffnungen entsteht vorteilhafterweise zwischen den kammerartigen Einrichtungen 5 und dem Körper 18 durch die strömende ionisierte Luft kein Staudruck und keine dadurch hervorgerufene Unebenheiten. Der Körper 18 besteht entweder aus Faserstoffen und ist ein Gewebe, ein Gewirk, ein Gestrick oder ein Vlies oder ist eine Folie aus Kunststoff oder besteht aus einem Verbundmaterial als Kombination der genannten Materialien. Die kammerartigen Einrichtungen 5 sind an der Decke des Raumes 4 befestigt. Der Körper 18 ist lösbar so gespannt, dass die Kammerwände mit den Durchbrüchen der kammerartigen Einrichtungen 5 lose entweder direkt am Körper 18 anliegen oder korrespondierend beabstandet zum Körper 18 angeordnet sind. Der Körper 18 besteht weiterhin aus einem schwer oder nicht entflammbaren Material.

Die Höhe der Ionisationsleistung des Ionisationsapparates 2, wobei die Ionisation durch elektrische Entladung an Ionisationsröhren oder an Koronaentladungsröhren erfolgt, wird in Abhängigkeit der Messungen
- insbesondere der Belastung der Außenluft mit flüchtigem Kohlenwasserstoff mit dem ersten Luftqualitätssensor 12,
- des Gehaltes an Ozon in der Zuluft mit dem Ozonsensor 13,
- der relativen Luftfeuchte der zu behandelnden Luft mit dem Luftfeuchtefühler 14,
- der Strömungsgeschwindigkeit oder des Volumenstroms der zu behandelnden Luft mit dem Luftströmungsfühler 15 und
- der oxidierbaren Luftbestandteile der Abluft und/oder Umluft mit dem zweiten Luftqualitätssensor 16 über die Steuereinrichtung 6 automatisch eingestellt.

Dazu werden in der Steuereinrichtung 6 die aus den Messungen gewandelten Signale von dem ersten Luftqualitätssensor 12, dem Ozonsensor 13, dem Luftfeuchtefühler 14, dem Luftströmungsfühler 15 und dem zweiten Luftqualitätssensors 16 so miteinander verknüpft, dass die Steuereinrichtung 6 eine situationsgerechte Leistung in Form von Wechselimpulsraten oder mehreren zu Paketen zusammengefassten Wechselimpulsraten an den Ionisationsapparat 2 abgibt, wenn eine höhere Luftmenge und/oder ein größere relative Luftfeuchte und/oder eine größere Raumluftbelastung mit flüchtigen Kohlenwasserstoffen - vaporous organic compounds (VOC) - oder ein größeres Oxidationspotenzial der Außenluft auftritt oder auftreten. Dabei erfolgt eine Vergrößerung der Wechselimpulsraten oder der Anzahl zu Paketen zusammengefasster Weehselimpulsraten. In der Steuereinrichtung erfolgt dazu
- eine Wichtung der einzelnen Parameter und eine Verknüpfung als Summe der einzelnen Parameter,
- eine Verknüpfung als Produkt aus den einzelnen Beträgen der Parameter oder
- eine andere mathematische Behandlung,
so dass der Ionisationsapparat 2 mit einer optimalen Leistung betrieben wird.

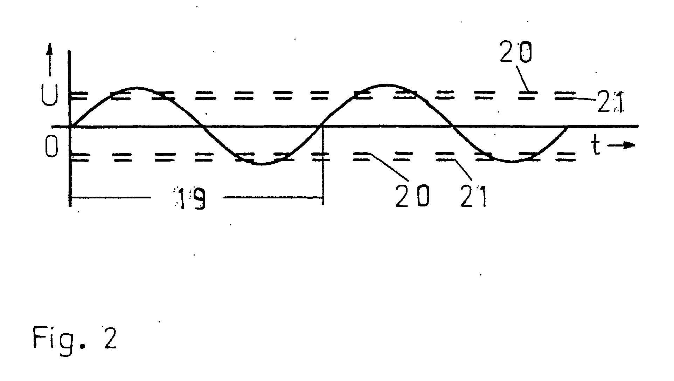

Der Ionisationsapparat 2 wird mit zeitlichen Folgen einer periodischen Wechselspannung gleicher oder annähernd gleicher Amplitude betrieben. Die kleinste Einheit der Folge ist dabei eine Periode der periodischen Wechselspannung als ein Wechselimpuls 19 (Darstellung in der Fig. 2).

Nichtbenötigte Perioden der periodischen Wechselspannung werden abgeführt. Dadurch wird gewährleistet, dass die Spannung bei der Entladung konstant bleibt. Die periodische Wechselspannung besitzt dabei eine Frequenz, die der jeweils bereitgestellten Frequenz des Netzes zur Energieversorgung entspricht, so dass ein Frequenzwandler nicht notwendig ist.

Eine stabile Luftionisation und damit eine optimale Wirkungsweise, dass heißt ein hoher Anteil von positiv und negativ geladenen Sauerstoffionen mit einem hohen Bindungsbestreben - z.B. mit dem VOC- Anteil der Luft und mit einem minimalen Anteil von Radikalen in der Luft, wird nur mit einer definierten Entladungsspannung erzeugt. Diese muss weitestgehend konstant gehalten werden, so dass ein Toleranzfeld eingehalten wird. Mit der Darstellung in der Fig. 2 wird nachfolgend das Verhalten der Entladung beim Ändern der Entladungsspannung beim Überschreiten der Grenze 20 und Unterschreiten der Grenze 21 desToleranzfeldes zwischen Grenzen 20, 21 einer optimalen Entladungsspannung beschrieben. Wird die Grenze 20 durch Anheben der Spannung des Ionisationsapparates 2 überschritten, wird progressiv die Ozonbelastung in der Zuluft ansteigen. Wird die Entladungsspannung dagegen unterhalb der Grenze 21 gesenkt, so ergibt sich ein Arbeitsfeld der Luftionisation, welche durch eine spontane Entladung (Puffereffekt) gekennzeichnet ist, wobei ebenfalls unerwünschte Sauerstoffradikale oder Ozon freigesetzt werden. Es wird daher eine definierte Entladungsspannung im Prozess konstant gehalten. Eine situationsgerechte und stabile Luftionisation wird durch eine entsprechende Aktivierung der im Nulldurchgang angeschnittenen Sinuskurve der definierten Wechselspannung erzielt. Dabei ist eine derartige Sinuskurve ein jeweiliger Wechselimpuls 19, der den Ionisationsapparat 2 in Funktion setzt. Zur weiteren Optimierung der Wirkungsweise der Luftionisation ist die Steuereinrichtung 6 so ausgelegt, dass zusätzlich die Wechselimpulsraten zu sinnvollen Paketen oder Mengen bestimmter Anzahl von Wechselimpulsen zusammengefasst werden können.

Die Signale des Ozonsensors 13 werden wie folgt ausgewertet oder im Prozess genutzt:
- von 0 bis 0,06 ppm Ozon-Anteil im Zuluftstrom keine Einflussnahme,
- größer/gleich 0,06 ppm Ozon-Anteil Absenkung der momentanen Ionisationsleistung auf 50 %,
- bei weiterem Anstieg des Ozon-Anteils liegt eine externe Ozonquelle vor und es werden die beschriebene Maßnahme zum Abbau des Ozons eingeleitet.
Der Betrieb erfolgt weiterhin so, dass ständig eine Ionisation erfolgt, auch wenn extrem niedrige Prozessdaten vorliegen, wobei der erste Luftqualitätssensor 12, der Ozonsensor 13, der Luftfeuchtefübler 14, der Luftströmungsfühler 15 und der zweite Luftqualitätssensor 16 signalisieren, dass an sich keine Ionisation erfolgen müsse. Dabei wird dem adäquaten Natureffekt entsprochen.

Während des Betriebs der Vorrichtung wird über die Fortluftleitung 10 nur eine geringe Menge an Fortluft abgeführt, der eine entsprechende Menge an Außenluft gegenübersteht, die dann über die Außenluftleitung 7 zugeführt wird. Damit ist eine gezielte Nutzung der Umluft zum Zweck der Energieeinsparung erreichbar.

Zur Optimierung der erforderlichen Energie ist vorteilhafterweise ein ständig angepasstes Verhältnis von Außenluft und Umluft möglich. Dieses Verhältnis ist unter anderem abhängig von der Außenlufttemperatur, des CO2-Gehaltes der Raumluft, Änderung der Raumtemperatur oder der Änderung der Raumenthalpie.

Das Stellelement des Reglers 17 für den Volumenstrom der Zuluft in der Zuluftleitung 8 vor der oder einer ersten kammerartigen Einrichtung 5 kann sowohl mit der Steuereinrichtung 6 und/oder einer weiteren Steuereinrichtung für die Raumtemperatur zusammengeschaltet sein. Im letzteren Fall kann eine Anordnung zur Beeinflussung und Behandlung der Zuluft vorteilhafterweise mit einer bereits installierten und damit vorhandenen Anordnung zum Temperieren der Raumluft zur erfindungsgemäßen Anordnung kombiniert werden.

Eine Deckenanordnung für eine erfindungsgemäße Anordnung zur Beeinflussung und Behandlung der Luft wenigstens eines Raumes 4 durch sowohl Temperieren als auch Ionisation der Zuluft besteht in einer Ausführungsform im wesentlichen aus mehreren in einer Ebene angeordneten und kammerartig ausgeführten Einrichtungen 5, einem flexiblen bahnenförmigen Körper 26 mit Öffnungen und einer Spann- und Befestigungseinrichtung zum lösbaren Spannen des flexiblen bahnenförmigen Körpers 26.

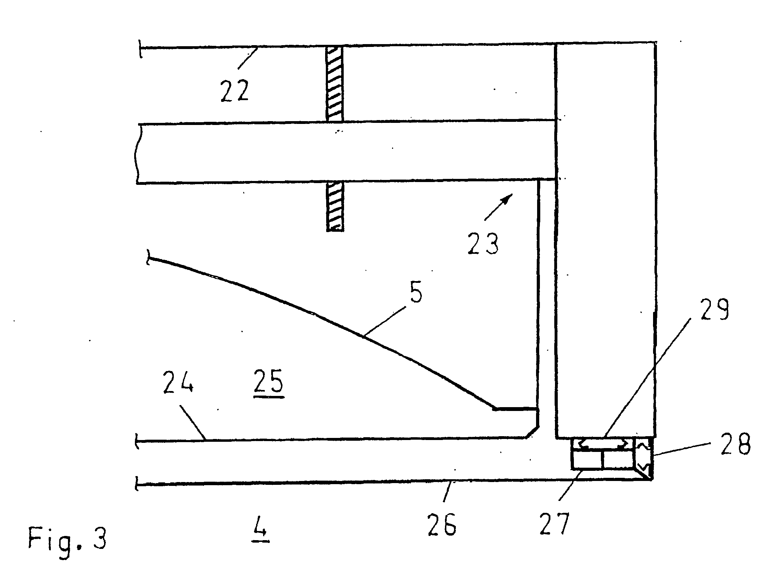

Die Fig. 3 zeigt eine Deckenanordnung für einen temperierbare Raum 4 und/oder für die Beeinflussung der Eigenschaften der Luft eines Raumes 4 in einer prinzipiellen Teilansicht.

Die kammerartigen Einrichtungen 5 sind mit Vorrichtungen 23 an der Decke 22 des Raumes 4 so befestigt, dass die Deckenfläche weitestgehend vollständig ausgefüllt ist. Derartige Vorrichtungen 23 sind bekannt, wobei in der Fig. 3 nur eine prinzipielle Darstellung gezeigt ist. Die in Richtung des Raumes 4 angeordnete Kammerwände 24 der kammerartigen Einrichtungen 5 weisen über die Fläche verteilt Durchbrüche auf Weiterhin besitzen diese Flächen festgelegte Abmessungen, so dass Räume 4 mit verschiedenen Flächen leicht mit diesen kammerartigen Einrichtungen 5 versehen werden können. Vorteilhafte Abmessungen sind dabei jeweils bis 60 x 60 cm². Die Hohlräume 25 der kammerartigen Einrichtungen 5 sind über Rohrleitungen miteinander verbunden, wobei eine Rohrleitung als Anschluss für alle kammerartigen Einrichtungen 5 nach außen endet. Dadurch können eine Vorrichtung zur Beeinflussung und Behandlung von Zuluft und/oder eine Vorrichtung zum Temperieren von Zuluft angeschlossen werden. Die kammerartigen Einrichtungen 5 bestehen vorzugsweise aus einem Metallblech oder einem Kunststoff.

Unter diesen kammerartigen Einrichtungen 5 befindet sich der flexible bahnenförmige Körper 26 in einer Spann- und Befestigungseinrichtung, der damit die Kammerwände 24 mit den Durchbrüchen überspannt.

Die Querschnitte der Durchbrüche als Öffnungen der kammerartigen Einrichtungen 5 sind kleiner oder gleich der Querschnitte der Öffnungen des flexiblen bahnenförmigen Körpers 26. Insbesondere bei gegenüber den Durchbrüchen größeren Öffnungen entsteht vorteilhafterweise zwischen den kammerartigen Einrichtungen 5 und dem flexiblen bahnenförmigen Körper 26 durch die strömende ionisierte Luft kein Staudruck und keine dadurch hervorgerufene Unebenheiten.

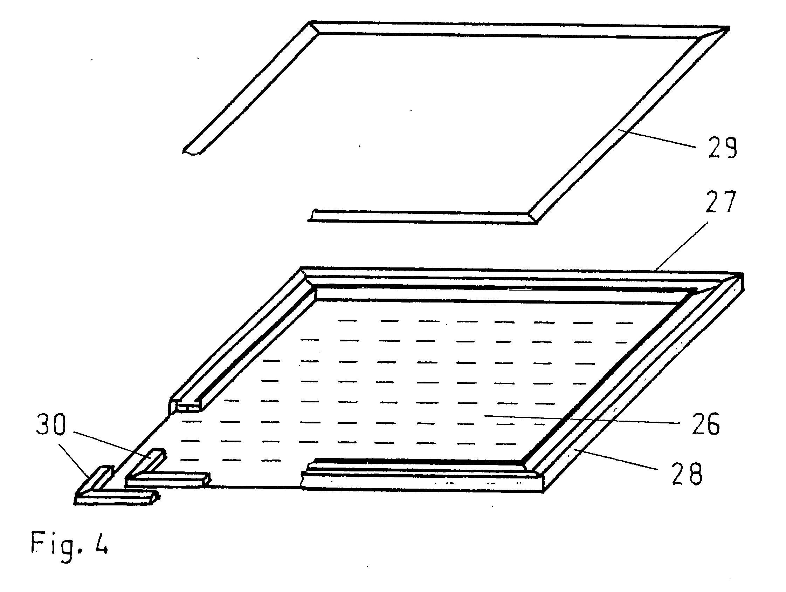

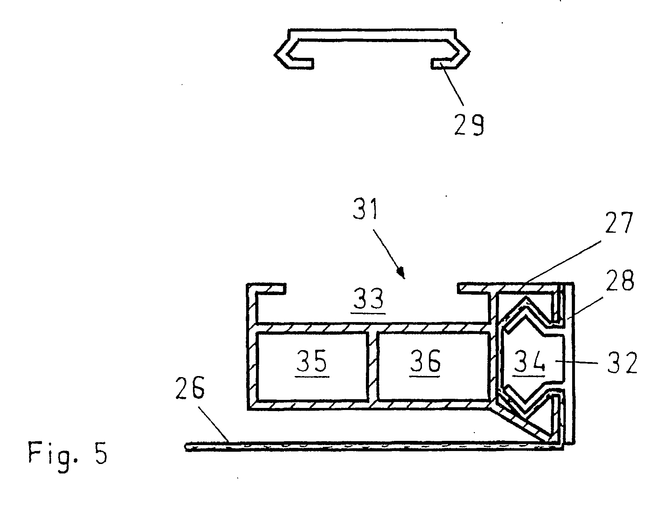

Für die Spann- und Befestigungseinrichtung sind mehrere Profilkörper 27 zu einen Rahmen angeordnet (Darstellung in der Fig. 4). Der Profilkörper 27 selbst besitzt vier durch drei Trennwände voneinander getrennte Hohlräume 33, 34, 35, 36 (Darstellung in der Fig. 5). Zwei rechtwinklig zueinander angeordnete Körperwände des Profilkörpers 27 weisen in Längsrichtung jeweils eine erste durchgehende Öffnung 31 und eine zweite durchgehende Öffnung 32 auf. Dadurch sind der erste Hohlraum 33 über die erste Öffnung 31 und der zweite Hohlraum 34 über die zweite Öffnung 32 von außen zugänglich. Die Breiten der ersten Öffnung 31 und der zweiten Öffnung 32 sind kleiner als die jeweilige Abmessung des ersten Hohlraumes 33 und zweiten Hohlraumes 34 jeweils in dieser Richtung (Darstellung in der Fig. 5). Der dritte Hohlraum 35 und der vierte Hohlraum 36 sind in ihren Unfang jeweils abgeschlossen. In den Endenbereichen der Profilkörper 27 sind die Endenbereiche von Eckelementen 30 platziert, so dass in einfacher Weise ein Rahmen realisierbar ist (Darstellung in der Fig. 4). Der Rahmen mit den Profilkörpern 27 ist so ausgeführt, dass die ersten Öffnungen in Richtung der Decke 22 des Raumes 4 und die zweiten Öffnungen 32 nach außen weisen.

In den zweiten Öffnungen 32 befindet sich ein erstes Einspreizmittel als Spannprofilkörper 28. Dieser besteht aus einem plattenförmigen Körper mit zwei beabstandeten Schenkeln. Jeder der Schenkel weist zwei winklig zueinander angeordnete Teilschenkel aus, wobei die Eckbereiche voneinander weg weisen. Der Abstand der Schenkel ist kleiner als die Breite der zweiten Öffnung 32 und der Abstand der winklig zueinander angeordneten Teilschenkel ist größer als die Breite der zweiten öffnung 32. Die Schenkel befinden sich weitestgehend im montierten Zustand des Spannprofilkörpers 28 im zweiten Hohlraum 34. Die Endenbereiche des flexiblen bahnenförmigen Körpers 26 befinden sich im gespannten Zustand an den Spannprofilkörpern 28 und bereichsweise zwischen Oberflächen der Profilkörper 27 und den Spannprofilkörpern 28. Die Längen des Spannprofilkörpers 28 und des Profilkörpers 27 sind vorzugsweise gleich. Die Fig. 5 zeigt unter anderem einen Profilkörper 27, einen flexiblen bahnenförmigen Körper 26 im gespannten Zustand und einen Spannprofilkörper 28 als Schnittdarstellung.

Im gespannten Zustand überspannt der flexible bahnenförmige Körper 26 die der Oberfläche mit den ersten Öffnungen 31 gegenüberliegenden Oberflächen der Profilkörper 27. Damit ist eine rahmenlose Fläche des flexiblen bahnenförmigen Körpers 26 für die Montage an Elementen an der Decke 22 des Raumes 4 vorhanden.

Die äußeren Bereiche der durch den flexiblen bahnenförmigen Körper 26 überspannten Oberflächen der Profilkörper 27 sind schräg angeordnet, wobei jeweils die Körperwand des Profilkörpers 27 mit der zweiten Öffnung 32 und der schräg angeordnete Wandbereich einen Winkel kleiner 90° einschließen. Damit ist sichergestellt, dass die die Profilkörper 27 überspannenden Bereiche des flexiblen bahnenförmigen Körpers 26 weitestgehend nicht an den Profilkörpern 27 anliegen.

In den ersten Öffnungen 31 und den ersten Hohlräumen 33 der Profilkörper 27 befinden sich zweite Einspreizelemente als Befestigungsprofilkörper 29. Diese Befestigungsprofilkörper 29 sind an der Decke 22 des Raumes 4 befestigten Elementen in Form des Rahmens befestigt, wobei die Abmessungen jeweils korrespondierend gleich sind. In den Fig. 4 und 5 sind der Rahmen mit dem flexiblen bahnenförmigen Körper 26 und die Befestigungsprofilkörper 29 voneinander getrennt dargestellt. Ein Befestigtmgsprofilkörper 29 besteht aus einem plattenförmigen Körper mit zwei beabstandeten Schenkeln. Jeder der Schenkel weist zwei winklig zueinander angeordnete Teilschenkel und einen parallel zum plattenförmigen Körper angeordneten dritten Teilschenkel auf, wobei die Eckbereiche des ersten und des zweiten Teilschenkels voneinander weg weisen. Der Abstand der Schenkel ist kleiner/gleich der Breite der ersten Öffnung 31 und der Abstand der winklig zueinander angeordneten Teilschenkel ist größer als die Breite der ersten Öffnung 31. Die Schenkel befinden sich im montierten Zustand des Befestigungsprofilkörpers 29 im ersten Hohlraum 33, so dass der plattenförmige Körper des Befestigungsprofilkörpers 29 und die Körperwandung des Profilkörpers 27 eine Ebene bilden. Der dritte Teilschenkel dient der Erhöhung der Stabilität des Befestigungsprofilkörpers 29.

Die Befestigung der Befestigungsprofilkörper 29 erfolgt varteilhafterweise mittels Schrauben. Die Einspreizmittel als Spannprofilkörper 28 und Befestigungsprofilkörper 29 bestehen vorzugsweise aus einem Kunststoff und der Profilkörper 27 aus einem Leichtmetall insbesonders Aluminium.

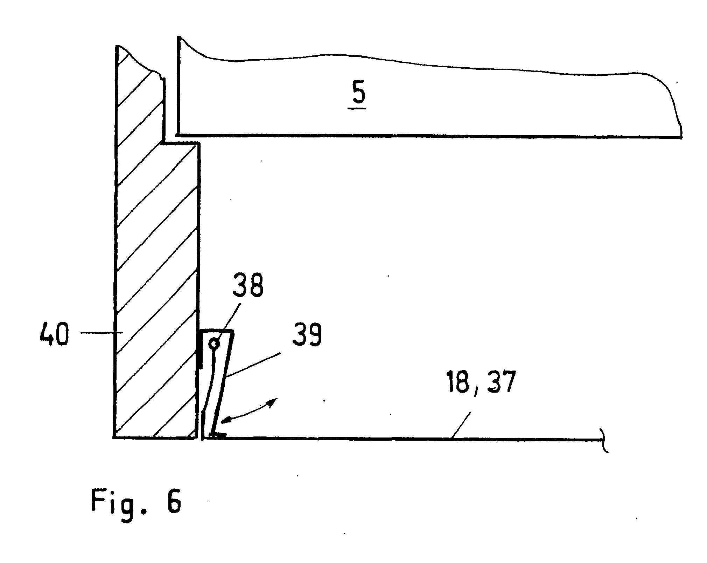

Eine weitere Spann- und Befestigungseinrichtung ist in einer weiteren Ausführungsform ein federnder Klemmmechanismus 39 (Darstellung in der Fig. 6). Dabei befindet sich der Endenbereich des Körpers 18 als eine bei Erwärmung sich ausdehnende Folie 37 aus Kunststoff
- im Klemmmechanismus 39 oder
- zwischen sowohl dem Klemmmechanismus 39 als auch einer Wand einer Rahmenanordnung 40 oder
- zwischen sowohl dem Klemmmechanismus 39 als auch einer Wand des Raumes 4.
Der Klemmmechanismus 39 ist vorteilhafterweise eine Federanordnung. Für eine leichte und einfache Platzierung der Endenbereiche der Folie 37 besitzt diese im Endenbereich eine Verdickung 38.

Die Rahmenanordnung 40 für die Folie 37 an der Decke des Raumes 4 kann auch den Abluftleitung 9 als Kanal aufnehmen, wobei Wandungen Einlassöffnungen für die Abluft des Raumes 4 aufweisen.

## Patentansprüche

1. Anordnung zur Beeinflussung und Behandlung der Luft wenigstens eines Raumes durch sowohl Temperieren als auch Ionisation der Zuluft mit folgenden Merkmalen:
- -einem ersten Luftqualitätssensor (12) in der Außenluftleitung (7) eines Luftaufbereitungsgerätes (1),
- -mindestens einem Ionisationsapparat (2), einem Ozonsensor (13), einem Luftfeuchtefühler (14), einem Luftströmungsfühler (15) und wenigstens einer Vorrichtung (3) zum Lufttemperieren in der Zuluftleitung (8) zwischen dem Luftaufbereitungsgerät (1) und dem wenigstens einem Raum (4),
- -mehreren in einer Ebene angeordneten kammerartigen, ein Bestandteil des Raumes (4) bildenden und mit der Zuluftleitung (8) gekoppelten Einrichtungen (5), wobei die in Richtung des Raumes (4) angeordneten Kammerwände Öffnungen als Durchbrüche aufweisen,
- -einem lose auf diesen Kammerwänden angeordnetem Körper (18) oder einem zu diesen Kammerwänden beabstandet angeordnetem Körper (18) mit die Konvektion der temperierten und durch Ionisation beeinflussten Zuluft in den Raum (4) gewährleistenden Öffnungen, wobei diese Kammerwände und der lose angeordnete Körper (18) die Wand oder die Decke als Zwischendecke und die Querschnitte der Durchbrüche als Öffnungen der kammerartigen Einrichtungen (5) kleiner oder gleich als die Querschnitte der Öffnungen des Körpers (18) sind,
- -sowohl einer nach außen endenden Fortluftleitung (10) als auch einer mit dem Luftaufbereitungsgerät (1) verbundenen Umluftleitung (11) an der Abluftleitung (9) des wenigstens einen Raumes (4),
- -einem zweiten Luftqualitätssensor (16) in der Umluftleitung (11) und
- -wenigstens einer mit dem ersten Luftqualitätssensor (12), dem Ozonsensor (13), dem Luftfeuchtefühler (14), dem Luflströmungsfühler (15), dem zweiten Luftqualitätssensor (16) und der Vorrichtung (3) zum Lufttemperieren zusammengeschalteten Steuereinrichtung (6).

2. Anordnung nach Patentanspruch 1, **dadurch gekennzeichnet, dass** in der Zuluftleitung (8) vor den kammerartigen Einrichtungen (5) oder einer der kammerartigen Einrichtungen (5) ein Regler (17) für den Volumenstrom der Zuluft eingeschalten ist und dass ein Antrieb für ein Stellelement des Reglers (17) für den Volumenstrom der Zuluft mit einer Steuereinrichtung und/oder einer Steuereinrichtung im Raum (4) und/oder mit der Steuereinrichtung (6) zusammengeschaltet ist.

3. Anordnung nach Patentanspruch 1, **dadurch gekennzeichnet, dass** die Hohlräume (25) der kammerartigen Einrichtungen (5) miteinander verbunden sind und dass ein mit Öffnungen versehener und **dadurch** die Konvektion von temperierter und/oder beeinflusster Zuluft ermöglichender flexibler bahnenförmiger Körper (26) mit einer Spann- und Befestigungseinrichtung die in Richtung des Raumes (4) angeordneten Kammerwände (24) der kammerartigen Einrichtungen (5) überspannt.

4. Anordnung nach Patentanspruch 3, **dadurch gekennzeichnet, dass** der flexible bahnenförmige Körper (26) entweder eine aus Kunststoffbestehende Folie ist oder aus Faserstoffen besteht und dass der flexible bahnenförmige Körper (26) ein Gewebe, ein Gewirk, ein Gestrick oder ein Vlies ist.

5. Anordnung nach Patentanspruch 1 oder 3, **dadurch gekennzeichnet, dass** der Körper (18) oder der flexible bahnenförmige Körper (26) aus einem schwer oder nicht entflammbaren Material besteht.

6. Anordnung nach Patentanspruch 1, **dadurch gekennzeichnet, dass** das eine die Höhe der Ionisationsleistung des Ionisationsapparates (2), wobei die Ionisation durch elektrische Entladung an Ionisationsröhren oder an Koronaentladungsröhren erfolgt, entsprechend der Messungen
- -insbesondere der Belastung der Außenluft mit flüchtigem Kohlenwasserstoff mit dem ersten Luftqualitätssensor (12),
- -des Gehalts an Ozon in der Zuluft mit dem Ozonsensor (13),
- -der relativen Luftfeuchte der zu behandelnden Luft mit dem Luftfeuchtefühler (14),
- -der Strömungsgeschwindigkeit oder des Volumenstroms der zu behandelnden Luft mit dem Luftströmungsfühler (15) und
- -der oxidierbaren Luftbestandteile der Abluft und/oder Umluft mit dem zweiten Luftqualitätssensor (16)
regelnde Steuereinrichtung (6) ist.

7. Anordnung nach Patentanspruch 6, **dadurch gekennzeichnet, dass** die Steuereinrichtung (6) und der Ionisationsapparat (2) so zusammengeschaltet sind, dass ständig Sauerstoffionen in der Zuluftleitung (8) vorhanden sind.

8. Anordnung nach Patentanspruch 6, **dadurch gekennzeichnet, dass** die Steuereinrichtung (6) und der Ionisationsapparat (2) so zusammengeschaltet ist, dass die Ionisationsleistung bei einem sich erhöhenden Anteil an flüchtigen Kohlenwasserstoffen und/oder der Luftgeschwindigkeit und/oder der relativen Luftfeuchte und/oder oxidierbaren Luftbestandteilen steigt.

9. Anordnung nach Patentanspruch 6, **dadurch gekennzeichnet, dass** die Steuereinrichtung (6) und der Ionisationsapparat (2) so zusammengeschaltet ist, dass bei Auftreten eines zu hohen Wertes an Ozon der Ozonwert durch Aufspaltung zurückgeführt wird, wobei der Ionisationsapparat (2) über eine zeitlich anliegende periodische Wechselspannung als mindestens ein Wechselimpuls, als Wechselimpulsrate oder als wenigstens ein Paket mit einer bestimmten Folge von Wechselimpulsen gesteuert ist.

10. Anordnung nach Patentanspruch 7, **dadurch gekennzeichnet, dass** die Steuereinrichtung (6) und der Ionisationsapparat (2) so zusammengeschaltet ist, dass bei einem Gehalt an Ozon in der Zuluftleitung (8) von größer/gleich 0,06 ppm die Leistung des Ionisationsapparates (2) gesenkt wird und dass bei weiteren Anstieg des Wertes an Ozon die Zeit der anliegenden periodischen Wechselspannung als Wechselimpulse, als Wechselimpulsrate und/oder Pakete mit Wechselimpulsen bestimmter Anzahl geändert wird.

## Claims

1. System for influencing and treating the air of at least one room by both tempering and ionizing the supply air, including
- a first air quality sensor (12) in the external air conduit (7) of an air conditioning device,
- at least one ionization apparatus (2), an ozone sensor (13), an air humidity sensor (14), an air flow sensor (15) and at least one device (3) for tempering the air in the supply conduit (8) between the air conditioning device (1) and the at least one room (4),
- several chamber-type devices (5) arranged in one plane, forming part of the room (4), coupled to the supply conduit (8), whereby the chamber walls arranged in direction of the room (4) are provided with openings as through-holes,
- a body (18) arranged unmounted on said chamber walls or a body (18) arranged distanced to said chamber walls, the body (18) provided with openings ensuring the convection of the tempered supply air into the room (4), said supply air being influenced using ionization, whereby said chamber walls and the body (18), which is arranged unmounted, are the wall or the ceiling as intermediate ceiling and the cross-sections of the through-holes as openings of the chamber-type devices (5) are smaller than or equal to the cross-sections of the openings of the body (18),
- both an exit air duct (10) ending to the outside and a recirculating air conduit (11), which is connected to the air conditioning device (1), at the exhaust air duct (9) of the at least one room (4),
- a second air quality sensor (16) in the recirculating air conduit (11), and
- at least one control device (6) in communicating connection with the first air quality sensor (12), the ozone sensor (13), the air humidity sensor (14), the air flow sensor (15), the second air quality sensor (16) and the device (3) for tempering the air.

2. System to claim 1 **characterized by that** in the supply conduit (8) upstream of the chamber-type devices (5) or one of the chamber-type devices (5), a controller (17) for the supply air volume rate is arranged and a drive for an actuating element of the controller (17) for the supply air volume rate is in communicating connection with a control device and/or a control device in the room (4) and/or the control device (6).

3. System to claim 1 **characterized by that** the hollow spaces (25) of the chamber-type devices (5) are connected with each other and a flexible, web-like body (26), which is provided with openings thus enabling the convection of tempered and/or influenced supply air, covers the chamber walls (24) arranged in direction of the room (4) of the chamber-type devices (5) by means of a tensioning and fastening device.

4. System to claim 3 **characterized by that** the flexible, web-like body (26) is either a film of a plastic material or is made of fibrous materials, and the flexible, web-like body (26) is a woven fabric, a knitted fabric or a non-woven.

5. System to claim 1 or 3 **characterized by that** the body (18) or the flexible, web-like body (26) is made of a flame-resistant or non-inflammable material.

6. System to claim 1 **characterized by that** the control device is a control device (6) that controls the level of the ionization output of the ionization apparatus (2), the ionization caused by electrical discharges at ionization tubes or corona-discharge tubes, according to measurements of
- particularly, the loading of the outside air by volatile hydrocarbons using the first air quality sensor (12),
- the ozone content in the supply air using the ozone sensor (13),
- the relative humidity of the air to be treated using the air humidity sensor (14),
- the flow velocity or volume flow rate of the air to be treated using the air flow sensor (15), and
- the oxidizable air constituents of the exhaust air and/or recirculating air using the second air quality sensor (16).

7. System to claim 6 **characterized by that** the control device (6) and the ionization apparatus (2) are in communicating connection such that oxygen ions are constantly present in the supply conduit (8).

8. System to claim 6 **characterized by that** the control device (6) and the ionization apparatus (2) are in communicating connection such that the ionization output increases with a rising volatile hydrocarbons proportion and/or air flow velocity and/or relative air humidity and/or oxidizable air constituents.

9. System to claim 6 **characterized by that** the control device (6) and the ionization apparatus (2) are in communicating connection such that when a too high ozone value occurs the ozone value is reduced by breaking, the ionization apparatus (2) being controlled through a periodic alternating voltage switched on in a timed manner as at least one alternating pulse, alternating pulse rate or at least one burst comprising a certain sequence of alternating pulses.

10. System to claim 7 **characterized by that** the control device (6) and the ionization apparatus (2) are in communicating connection such that for an ozone content in the supply conduit (8) of higher than/equal to 0.06 ppm the output of the ionization apparatus (2) is decreased and when the ozone value further increases, the time of the periodic alternating voltage applied as alternating pulses, alternating pulse rate and/or bursts comprising a certain sequence of alternating pulses is changed.

## Revendications

1. Dispositif pour influencer et traiter l'air d'au moins une pièce par régulation de la température ainsi que par ionisation de l'air amené, possédant les caractéristiques suivantes :
- un premier capteur de qualité d'air (12) dans la conduite d'air extérieur (7) d'un appareil de traitement d'air (1),
- au moins un appareil d'ionisation (2), un capteur d'ozone (13), un capteur d'humidité d'air (14), un capteur d'écoulement d'air (15) et au moins un dispositif (3) de régulation de la température d'air dans la conduite d'amenée d'air (8) entre l'appareil de traitement d'air (1) et ladite au moins une pièce (4),
- plusieurs dispositifs (5) du type chambre, disposés dans un plan, formant un élément de la pièce (4) et couplés à la conduite d'amenée d'air (8), les parois de chambres disposées en direction de la pièce (4) présentant des ouvertures sous forme de traversées,
- un corps (18) disposé de manière lâche sur ces parois de chambres ou un corps (18) disposé à distance de ces parois de chambres avec des ouvertures assurant la convection de l'air tempéré et influencé par ionisation dans la pièce (4), ces parois de chambres et le corps (18) disposé de manière lâche étant le mur ou le plafond sous forme de faux-plafond et les sections des traversées sous forme d'ouvertures des dispositifs (5) du type chambre étant inférieures ou égales aux sections des ouvertures du corps (18),
- aussi bien une conduite d'évacuation d'air (10) aboutissant à l'extérieur qu'une conduite de recirculation d'air (11) reliée à l'appareil de traitement d'air (1) sur la conduite de sortie d'air (9) de ladite au moins une pièce (4),
- un deuxième capteur de qualité d'air (16) dans la conduite de recirculation d'air (11) et
- au moins un dispositif de commande (6) interconnecté avec le premier capteur de qualité d'air (12), le capteur d'ozone (13), le capteur d'humidité d'air (14), le capteur d'écoulement d'air (15), le deuxième capteur de qualité d'air (16) et le dispositif (3) pour la régulation de température.

2. Dispositif selon la revendication 1, **caractérisé en ce qu'**un régulateur (17) pour le débit volumique de l'air amené est connecté dans la conduite d'amenée d'air (8) en amont des dispositifs (5) du type chambre ou d'un des dispositifs (5) du type chambre et qu'un entraînement pour un élément de commande du régulateur (17) pour le débit volumique de l'air amené est interconnecté avec un dispositif de commande et/ou un dispositif de commande dans la pièce (4) et/ou avec le dispositif de commande (6).

3. Dispositif selon la revendication 1, **caractérisé en ce que** les cavités (25) des dispositifs (5) du type chambre sont reliés entre elles et qu'un corps flexible en forme de feuille (26) pourvu d'ouvertures et permettant donc la convection d'air amené tempéré et/ou influencé recouvre avec un dispositif de tension et de fixation les parois de chambres (24) disposées en direction de la pièce (4) des dispositifs (5) du type chambre.

4. Dispositif selon la revendication 3, **caractérisé en ce que** le corps flexible en forme de feuille (26) est soit une feuille en matière plastique, soit composé de matières fibreuses et que le corps flexible en forme de feuille (26) est un tissu, un tissu à mailles, un tricot ou un non-tissé.

5. Dispositif selon la revendication 1 ou 3, **caractérisé en ce que** le corps (18) ou le corps flexible en forme de feuille (26) est composé d'un matériau difficilement ou non inflammable.

6. Dispositif selon la revendication 1, **caractérisé en ce que** le dispositif de commande est un dispositif de commande (6) régulant le niveau de la puissance d'ionisation de l'appareil d'ionisation (2), l'ionisation étant réalisée par décharge électrique sur des tubes à ionisation ou sur des tubes à décharge corona, en fonction des mesures
- en particulier de la charge de l'air extérieur en hydrocarbure volatil avec le premier capteur de qualité d'air (12),
- de la teneur en ozone dans l'air amené avec le capteur d'ozone (13),
- de l'humidité relative de l'air de l'air à traiter avec le capteur d'humidité d'air (14),
- de la vitesse d'écoulement ou du débit volumique de l'air à traiter avec le capteur d'écoulement d'air (15) et
- des composants oxydables de l'air dans l'air de sortie et/ou l'air de recirculation avec le deuxième capteur de qualité d'air (16).

7. Dispositif selon la revendication 6, **caractérisé en ce que** le dispositif de commande (6) et l'appareil d'ionisation (2) sont interconnectés de façon que des ions oxygène soient présents en permanence dans la conduite d'amenée d'air (8).

8. Dispositif selon la revendication 6, **caractérisé en ce que** le dispositif de commande (6) et l'appareil d'ionisation (2) sont interconnectés de façon que la puissance d'ionisation augmente en cas d'augmentation de la proportion d'hydrocarbures volatils et/ou de la vitesse de l'air et/ou de l'humidité relative et/ou de composants oxydables de l'air.

9. Dispositif selon la revendication 6, **caractérisé en ce que** le dispositif de commande (6) et l'appareil d'ionisation (2) sont interconnectés de façon qu'en cas de survenue d'une valeur trop élevée d'ozone, la valeur d'ozone est réduite par décomposition, l'appareil d'ionisation (2) étant commandé par une tension alternative périodique appliquée temporellement sous la forme d'au moins une impulsion alternative, d'un taux d'impulsions alternatives ou d'au moins un paquet contenant une séquence déterminée d'impulsions alternatives.

10. Dispositif selon la revendication 7, **caractérisé en ce que** le dispositif de commande (6) et l'appareil d'ionisation (2) sont interconnectés de façon qu'en cas de teneur en ozone dans la conduite d'amenée d'air (8) supérieure/égale à 0,06 ppm, la puissance de l'appareil d'ionisation (2) est diminuée et qu'en cas d'augmentation supplémentaire de la valeur d'ozone le temps de la tension alternative périodique appliquée sous forme d'impulsions alternatives, de taux d'impulsions alternatives et/ou de paquets d'impulsions alternatives en nombre déterminé est modifié.
